# EUROPEAN PATENT SPECIFICATION

(11) **EP 4 181 798 B1**
(45) Date of publication and mention of the grant of the patent: **29.10.2025**
(21) Application number: 21859301.0
(22) Date of filing: 17.08.2021
(51) Int. Cl.: A61B 17/12, A61B 17/00, A61B 17/22, A61B 17/42, A61F 2/01, A61F 2/06, A61M 29/00

(54) **VASCULAR EMBOLIC IMPLANT**
VASKULÄRES EMBOLIEIMPLANTAT
IMPLANT EMBOLIQUE VASCULAIRE

(30) Priority: 18.08.2020 US 202063067016 P
(43) Date of publication of application: 24.05.2023
(73) Proprietor: BALT USA, LLC, Irvine, CA 92618 (US)
(72) Inventor: LE, Jake, Irvine, California 92618 (US)
(74) Representative: Marks & Clerk LLP
(86) International application number: PCT/US2021/071217
(87) International publication number: WO 2022/040683

(56) References cited:
- EP-A2- 1 867 289
- US-A1- 2005 090 855
- US-A1- 2008 228 216
- US-A1- 2013 018 409
- US-A1- 2017 086 852
- US-A1- 2018 271 533
- US-A1- 2019 298 387

## Description

### RELATED APPLICATIONS

This application claims priority to U.S. Provisional Application No. 63,057,016.

### BACKGROUND

### Field

This application relates generally to medical devices, and more specifically, to apparatuses, systems and methods for the treatment of aneurysms and other vascular abnormalities.

### Description of the Related Art

Implantable medical devices exist for treating a number of diseases and conditions associated with body lumens. Occlusive devices can be used to at least partially fill or occupy certain vascular or other anatomical spaces (e.g., vascular aneurysms). Vascular aneurysms, for example, which can develop as a result of a weakening in the arterial wall, can lead to internal bleeding. Occlusion of aneurysms and other such spaces using implant devices can be used to treat such potentially dangerous and lethal conditions.

US2017086852 describes an embolization device for treating ischemic stroke. The embolization device has a surface, wherein a body portion is configured to have a radiopaque and electropositive surface under physiological conditions when the device is emplaced, which ionically binds a blood component in an amount effective to promote stability of device in situ to bind to a tissue component in an amount effective to increase adhesion of the device as compared to a device without an electropositive surface. Embolic coils being so delivered are electrolytically detachable in under 10 seconds, according to the disclosed vascular implant systems.

US2019298387 describes an implant for treatment of a vascular space, The implant forms open loops and closed loops in separate cycles to provide balanced stiffness and flexibility. Such a configuration can provide proper coverage over an opening to a vascular space into which the implant is delivered, for reducing or eliminating flow into or out of the body cavity and promoting occlusion of the vascular space.

### Summary

The present invention is defined in claim 1. Some preferred features are defined in the dependent claims.

According to some embodiments, a vascular embolic implant (e.g., a vaso-occlusive implant or device) comprises a coil configured to assume an elongated or substantially straight or linear shape for delivery into a subject and configured to assume an implanted or non-elongated (e.g., expanded or three-dimensional) shape when positioned into a targeted vascular defect of the subject, wherein the coil is configured to form six sides when in the non-elongated (e.g., expanded, three-dimensional or non-linear) shape, wherein at least one side formed by the implanted coil comprises a large loop, and wherein at least one side formed by the implanted coil comprises two or more small loops.

According to some embodiments, a diameter of the large loop is at least 70% of a width of a corresponding side of the implant. In some embodiments, the diameter of the large loop is at least 75% of the width of the corresponding side of the implant. In some embodiments, the diameter of the large loop is at least 80% of the width of the corresponding side of the implant.

According to some embodiments, a diameter of each of the small loops is less than 50% of a width of a corresponding side of the implant. In some embodiments, the diameter of each of the small loops is less than 40% of the width of a corresponding side of the implant. In some embodiments, the diameter of each of the small loops is less than 30% of the width of a corresponding side of the implant. In some embodiments, the diameter of each of the small loops is less than 20% of the width of a corresponding side of the implant.

According to some embodiments, the implant comprises three sides that include a large loop and three sides that include two or more small loops. In other embodiments, the implant comprises two sides that include a large loop and four sides that include two or more small loops, or vice versa. In other embodiments, the implant comprises one side that includes a large loop and five sides that include two or more small loops, or vice versa.

According to some embodiments, the coil is configured to transition from a first side to an immediately adjacent side for all sides of the implant. In some embodiments, the transition includes a 90-degree transition to the adjacent side. In some embodiments, a six-sided implant includes a coil that comprises five sequential immediately-adjacent (e.g., 90-degree or substantially 90-degree) transitions. According to some embodiments, two or more of the sides of an implant can be configured to daisy chain onto a single side. In some embodiments, such a configuration can increase the length of the implant.

According to some embodiments, the at least one side comprising two or more small loops comprises at least three loops. In some embodiments, the at least one side comprising two or more small loops comprises at least four loops (e.g., 4, 5, more than 5, etc.).

According to some embodiments, the coil comprises a shape memory material. In some embodiments, the shape memory material comprises a platinum tungsten alloy.

According to some embodiments, the coil is configured to be delivered to the targeted vascular defect of the subject using a delivery tool. In some embodiments, the delivery tool comprises a pusher member and a tether member.

According to some embodiments, the tether member is configured to be releasable secured to a portion of the coil. In some embodiments, the tether member is configured to be compromised to release the tether member from the coil after the implant has been delivered to the targeted vascular defect of the subject. In some embodiments, the tether member is configured to be compromised mechanically, thermally, chemically or electrolytically.

### Brief Description of the Drawings

These and other features, aspects and advantages of the present application are described with reference to drawings of certain embodiments, which are intended to illustrate, but not to limit, the present disclosure It is to be understood that these drawings are for the purpose of illustrating the various concepts disclosed herein and may not be to scale.
FIG. 1 illustrates a perspective view of a vaso-occlusive device according to one embodiment;
FIG. 2 is a different perspective view of the vaso-occlusive device of FIG. 1;;
FIGS. 3 and 4 illustrate different perspective views of sides of the vaso-occlusive device of FIGS. 1 and 2;
FIGS. 5A to 5F illustrate different planar views of the various sides of the device of FIGS. 1 and 2;
FIG. 6 schematically illustrates an implant releasably secured to a delivery device according to one embodiment;
FIG. 7 illustrates a coil or member of an implant in a wound orientation; and
FIGS. 8A and 8B illustrate side and cross-sectional view of an embodiment of a coil that comprises an implant.

### Detailed Description

Although the various embodiments of a handpiece assembly have specific relevance to vaso-occlusive devices to treat cerebral aneurysms and other vascular abnormalities, the features, advantages and other characteristics disclosed herein may have direct or indirect applicability in other applications, such as, for example, medical devices, mechanical devices and/or the like.

Several embodiments of the disclosure are particularly advantageous because they include one, several or all of the following benefits: provide for an vaso-occlusive implant device with an enhanced shape, provide a vaso-occlusive implant device having greater strength properties, the implant is intended to have enhanced shape memory properties, e.g., the implant having the necessary stiffness such that it can, more predictably, reliably and in an improved manner, form a basket in the aneurysm to its pre-determined shape. In some embodiments, the implant is shaped, designed and otherwise configured to have more loops for increased neck coverage. In some configurations, the smaller loops of the implant combined with the enhanced shape memory properties can result in an improved covering of the targeted anatomical area (e.g., neck or other portion of the aneurysm.

The treatment of intracranial and/or other aneurysms with the use of microcoils and/or other implants has certain benefits over other approaches (e.g., surgical clipping). In some embodiments, a microcatheter or other intraluminal device is advanced within the subject so that the tip is positioned adjacent the neck of the aneurysm. Subsequently, a microcoil or other implant can be delivered through the lumen of the microcatheter or other intraluminal device into the aneurysm or other vascular defect.

The present application discloses various embodiments of a microcoil device or other implant and the manner in which it is delivered into an aneurysm or other defect. The shape, size, configuration and/or other details regarding the implant can help ensure that the implant remains within the desired aneurysm following implantation.

The microcoil and/or other implant embodiments disclosed herein can be used with any size defect and/or to treat any indication, as desired or required. Further, as noted above, the implants can be delivered and released within the target anatomy of a subject using any desired protocol or technique.

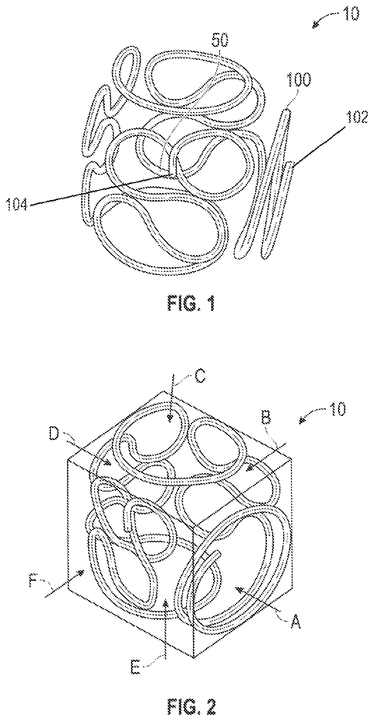

FIG. 1 illustrates one embodiment of a vaso-occlusive implant 10 comprising a generally three-dimensional shape upon release into the subject. As shown, the implant 10 includes an intricate outer shape formed by a coil member 100. The shape of the coil member 100 can define an interior portion or cavity 50.

With continued reference to the embodiment of FIG. 1, the implant 10 can include a generally cubic shape in which the implant includes six sides. As shown, the implant, in its implanted or non-elongated (e.g., expanded, three-dimensional or non-linear) shape, can include a plurality of rings. In some embodiments, the expandable member or portion 100 that forms the rings can be orientated generally (e.g., substantially) along one of the six sides of a cube or other three-dimensional six-sided device. In some embodiments, the implant has a generally or substantially cubic shape wherein all six sides are equal or substantially equal in cross-sectional shape. For example, in some arrangements, each of the "sides" of the implant 10 are approximately within 100% to 125% (e.g., 100 to 125, 100 to 105, 105 to 110, 110 to 115, 115 to 120, 120 to 125, 100 to 110, 110 to 120, 115 to 125%, percentages between the foregoing ranges and values, etc.) of the area of any other side.

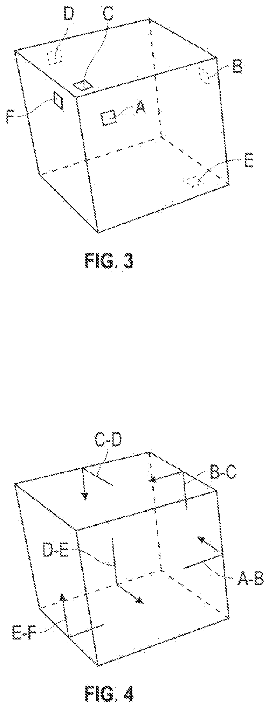

As illustrated (e.g., schematically) in FIG. 2, each of the sides (e.g., represented by perpendicular or normal arrows A, B, C, D, E, F) are offset at or near 90 degrees relative to one another in order to form a six-sided cubic shape. For clarity, the different "sides" A, B, C, D, E, F of the implant 10 are illustrated schematically in FIG. 3.

The implant 10 can comprise a single member 100 that is configured to assume the depicted shape upon release into the anatomy of a subject. The member 100 can include a helical member that includes a platinum tungsten alloy. For example, in some embodiments, the platinum tungsten alloy comprises an alloy of 92% platinum, 8% tungsten (92/8 Pt/W), other alloys and/or other shape memory material having superelastic properties. However, in some embodiments, the member 100 can include a non-helical shape and/or materials that are not shape memory materials, as desired or required.

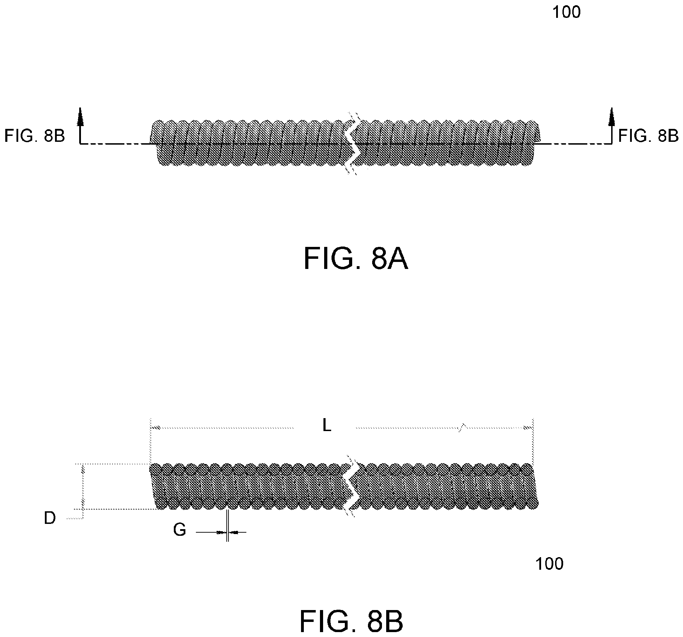

According to some embodiments, the member 100 comprises inner or core wire member which is surrounded by an outer wire member. In some embodiments, the outer wire member is wound (e.g., helically) around the outside of the core wire. In some arrangements, one or more exterior layers or coatings can be positioned along the outside of both the outer wire member and the core wire. According to some embodiments, as illustrated in FIGS. 8A and 8B, the member 100 can include a helically-wound member that comprises sequential turns or wounds. In some embodiments, such turns or wounds are angled relative to an longitudinal and/or radial axis of the member 100. For instance, the member can include turns or wounds that are angled 0 to 90 degrees (e.g., 0 to 90, 10 to 70, 20 to 60 degrees, angles between the foregoing, etc.) relative to the longitudinal axis and/or the radial axis of the member 100, as desired or required. In some embodiments, the member can include only a single alloy or other member that is wound about an axis. However, in other configurations, as noted above, the member 100 can include an inner member and/or one or more outer members, coatings and/or coverings.

With continued reference to FIGS. 8A and 8B, the length L, diameter or other cross-sectional dimension D and/or the size of a gap G between sequential turns or wounds G can be modified to accommodate a particular design and/or application or use. In some embodiments, the diameter or other cross-sectional dimension D of the member 100 is 0.127mm to 1.27mm (0.005 to 0.050 inches), e.g. 0.127mm to 1.27mm, 0.254mm to 0.381mm, 0.2032mm to 0.889mm, 0.3175mm to 0.08255mm (0.005 to 0.050, 0.010 to 0.015, 0.008 to 0.035, 0.0125 to 0.00325 inches), values between the foregoing, etc.

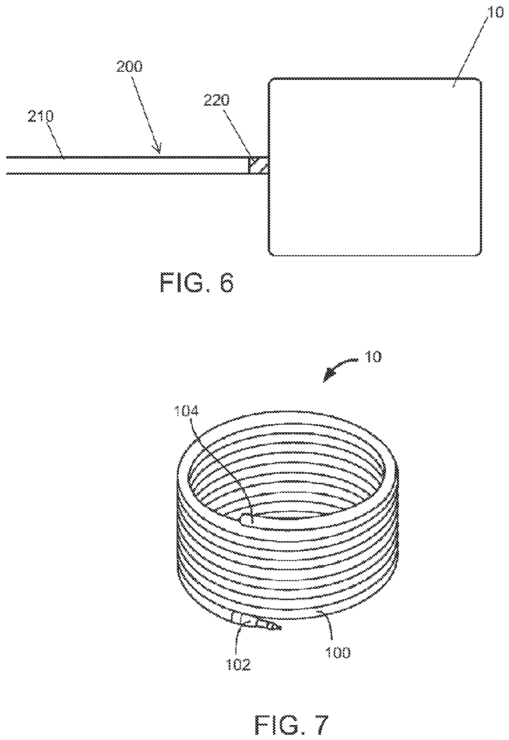

According to some embodiments, the implant 10 is configured to assume a wound shape as illustrated in FIG. 7. In some arrangements, the member 100 of the implant is wound on a single cylindrical mandrel (not shown). The member 100 of the implant 10 can be formed from an embolic coil member 100 having a first end 102 and a second end 104.

With continued reference to FIGS. 1 and 2, as noted above, the implant 10 can include six sides (e.g., A to F) that define an interior region 50. In some embodiments, the implant 10 comprises a single coil or member 100 that extends from a first end 102 to a second end 104. As shown in FIGS. 1 and 2, and highlighted schematically by arrows in FIG. 4, the coil or member 100 can form one or more desired shapes along each "side" of the implant 10 before transitioning to an adjacent "side" of the implant.

In some embodiments, as depicted schematically in FIG. 4, the coil or other member 100 of the device is configured to transition from one "side" to an adjacent "side" of the implant 10 when in the implanted or non-elongated (e.g., expanded) configuration. As shown, the transitions between sides can include transitions to an immediately adjacent side (e.g., one that is offset by approximately 90 degrees relative to the first side). Thus, as shown, each of the member's 100 transitions A to B, B to C, C to D, D to E, E to F can be to an immediately adjacent side. In other configurations, however, the transitions can occur in a different manner (e.g., from one "side" to a "side" that is not immediately adjacent), as desired or required.

As illustrated in FIGS. 1 and 2, the implant, in its implanted or non-elongated (e.g., expanded or non-linear) form, can include one or more loops, curves and/or other shapes along each side. Accordingly, the coil or other member 100 of the implant 10 can be configured to assume a desired configuration when implanted or non-elongated (e.g., expanded or not radially constrained). The loops or other shapes formed by the coil or member can vary along one or more sides of the implant. Alternatively, the coil or other member 100 of the implant can form similar loops or shapes along two or more of the sides, as desired or required.

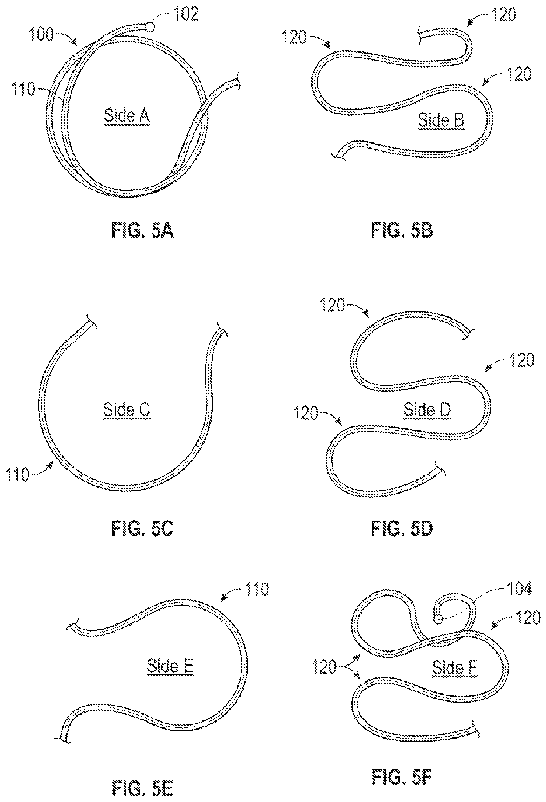

FIGS. 5A to 5F illustrate front views of each of the six sides of the implant embodiment of FIGS. 1 and 2. As shown, some of the sides, such as, for example, Sides A, C and E, are characterized by the coil or member 100 forming larger loops or curves 110. However, other sides, such as, for example, Sides B, D and F includes two or more (e.g., 2, 3, 4, more than 4, etc.) smaller loops or curves 120.

According to some embodiments, the configurations disclosed herein can result in greater neck or other coverage for a targeted aneurysm. Further, the increased or enhanced shape memory characteristics resulting from the expanded (e.g., non-linear) shape of the implant can improve basket formation to a pre-determined shape.

Therefore, as illustrated herein, a six-sided implant 10 can include three sides that comprise larger loops 110 and three sides that comprise smaller loops 120. In some embodiments, each side (e.g., Side A, C and E) that is formed by a larger loop 110 can include only a single loop 110. Further, in some embodiments, each side (e.g., Side B, D and F) that is formed by smaller loops 120 can include a plurality of loops 120.

In alternative arrangements, however, sides with larger loops 110 can include more than one loop 110. Likewise, sides with smaller loops 120 can include only a single loop or can include a plurality of loops 120. Therefore, a side can include one, two, three, four or more larger or smaller loops 110, 120, as desired or required for a particular application or use.

In some embodiments, a larger loop 110 is one that includes a diameter (e.g., along the loop or curve) that is at least 70% to 80% of the width or other cross-sectional dimension of the side in which the loop or curve 110 is located. Further, in some arrangements, a smaller loop 120 is one that includes a diameter (e.g., along the loop or curve) that is less than 50% (e.g., less than 50%, less than 45%, less than 40%, less than 35%, less than 30%, less than 25%, less than 20%, etc.) of the width or other cross-sectional dimension of the side in which the loop or curve 120 is located.

In the illustrated embodiments, the implant 10 comprises three sides (Sides A, C and E) with larger loops or curves 110 and three sides (Sides B, D and F) with smaller loops or curves 120. However, in other arraignments, an implant 10 comprises more or fewer sides with larger loops or curves 110 and/or more or fewer sides with smaller loops or curves 120, as desired or required. For instance, in one embodiment, an implant 10 can comprise one or two sides with larger loops or curves 110 and five or four sides with smaller loops or curves 120, or vice versa.

As shown schematically in FIG. 6, the vaso-occlusive device or implant 10 can include a tether 220 constructed from one or more of a variety of materials, including, for example, one or more thermoplastic elastomers. The tether 220 can be included as part of a delivery device 200 and located along the distal end of a pusher member 210 of such a device 200. In some embodiments, the implant 10 is detachably coupled to the pusher member 210 of a delivery device 200 via a coupling joint or tether 220.

In some arrangements, the tether 220 comprises a thermoplastic elastomer, such as, for example, Engage^{®}, a polyester strand (e.g., polyethylene terephthalate (PET)) and/or any other material. The diameter of the tether 220 can be 19.05µm to 76.2µm (0.00075 inches to 0.0030 inches), e.g. 38.1µm to 76.2µm, 50.8µm to 63.5µm, 19.05µm to 38.1µm, 25.4µm, 55.88µm (0.0015 to 0.0030, 0.0020 to 0.0025, 0.00075 to 0.0015, 0.0010, 0.0022 inches), values between the foregoing ranges, etc.

The coil or other member 100 of the implant 10 can comprise a diameter or other cross-sectional dimension of 0.254mm to 0.762mm (0.010 inches to 0.030inches), e.g. 0.254mm to 0.762mm, 0.254mm to 0.508mm, 0.254mm to 0.381 mm, 0.2794mm to 0.3302mm, 0.3048mm, etc. (0.010 to 0.030 inches, 0.010 to 0.020 inches, 0.010 inches to 0.015, 0.011 to 0.013 inches, 0.012 inches, etc.). The length (when straight) of the coil or other member 100 can be 0.5 cm to 100 cm (e.g., 0.5 to 100, 0.5 to 75, 0.5 to 50, 1 to 40 cm, values between the foregoing, etc.), as desired or required.

According to some embodiments, delivery and deployment of the implant device 10 can be carried out by first compressing the implant device 10 for intravascular delivery through the subject. While disposed within a microcatheter or other intraluminal device, the implant's filamentary elements of layers may take on an elongated, non-everted configuration substantially parallel to each other and to a longitudinal axis of the microcatheter.

In some embodiments, once the implant device 10 is pushed out of the distal end of the microcatheter or other intraluminal device (or a radial constraint is otherwise removed relative to the wire or member 100 of the device 10), the distal ends of the filamentary elements may then axially contract towards each other, so as to assume a three-dimensional configuration within the aneurysm or other vascular defect. Any other method or technique can be used, however, to deliver the implant 10 to a desired anatomical location of the subject, as desired or required. The distal end of the microcatheter or intraluminal device can be positioned such that a distal port of the microcatheter is directed towards or disposed within a vascular defect (e.g., aneurysm) A to be treated and the delivery member (e.g., delivery wire) withdrawn. The implant device 10 secured to the delivery apparatus can be inserted (e.g., while in an elongated or substantially straight, linear or constrained orientation) into a proximal portion of the inner lumen of the microcatheter or other intraluminal device.

The implant 10 can then be advanced distally to the vascular defect through the inner lumen of the delivery device. In some embodiments, once the distal tip or deployment port of the delivery system is positioned in a desirable location adjacent or within a vascular defect, the implant device 10 may be deployed out of the distal end of the microcatheter, thus allowing the device to begin to assume a three-dimensional or implanted shape. As the implant device 10 emerges from the distal end of the delivery apparatus, and the microcatheter, the implant device 10 may start to assume an non-elongated or implanted (e.g., non-linear) state within the vascular defect. In some embodiments, to release the implant 10 within the aneurysm or other vascular defect, the implant device 10 can be detached from the delivery apparatus by compromising the tether. Such detachment can be accomplished using any known method, such as using heat or electrical energy to melt the tether, cutting or otherwise mechanically compromising the tether, chemically compromising the tether and/or the like.

As noted above, the microcoils and/or other implants disclosed herein can be detachable for purposes of delivery and implantation into a desired anatomical location. Any of a variety of known detachment methods or techniques can be used to deliver the implant within a desired anatomical location, as desired or required. Though detachment systems can include some dynamic process, some systems involve more physical movement of the system than others. For example, mechanical detachment systems, using pressure, unscrewing, axial pistoning release and/or the like tend to cause a finite amount of movement of the implant at the aneurysm during detachment.

In some arrangements, non-mechanical detachment systems (e.g., chemical, temperature, electrolytic, etc.) may include less movement. However, in some arrangements, such systems often suffer from less consistency. For example, a consistent short duration for a coil to detach can be experienced when using such systems. Though electrical isolation of the implant coil itself has aided in lower average coil detachment times, there is still some inconsistency in how quickly the coils will detach. In a larger aneurysm that might have ten or more coils implanted, the large or unpredictable detachment times are multiplied, and delay the procedure. Additionally, a single large detachment time may risk instability during the detachment, due to movement of the patient of the catheter system. Even systems that indicate that detachment has occurred, for example by the measurement of a current below a certain threshold, are not completely trusted by users.

A number of detachable systems include a particular structure at a junction between a pusher wire and the detachable coupled microcoil implant. Of course, the delivery system and/or the implant can be sized, shaped and otherwise configured to permit for safe, predictable and reliable detachment to occur.

In some embodiments, one or more implants 10 can be positioned within a targeted aneurysm or other vascular defect. In some arrangements, the implant(s) can be used to fill or otherwise occupy a desired volume of the aneurysm. For example, the size, shape, quantity and/or details of the implant(s) can be selected to fill as much volume as possible of a targeted defect. In some arrangements, 80% to 100% of a targeted defect can be filled by the implant(s). However, in other embodiments, less than 80% of the defect may be filled by the implant(s). The comparatively soft nature of the implants 10 allows a sufficient amount of packing to achieve a desired level of thrombosis and occlusion, without creating potentially dangerous stresses on the wall of the aneurysm that could potentially least to rupture (or re-rupture).

Although several embodiments and examples are disclosed herein, the present application extends beyond the specifically disclosed embodiments to other alternative embodiments and/or uses of the modifications and equivalents thereof. It is also contemplated that various combinations or subcombinations of the specific features and aspects of the embodiments may be made and still fall within the scope of the disclosure. Accordingly, it should be understood that various features and aspects of the disclosed embodiments can be combined with or substituted for one another in order to form varying modes of the disclosure. Thus, it is intended that the scope of the present invention should not be limited by the particular disclosed embodiments described above, but should be determined only by a fair reading of the claims that follow.

## Claims

1. A vaso-occlusive implant (10) comprising:
a coil member (100) expandable from an elongated shape to an implanted shape, the elongated shape for delivery into a subject, and the coil member (100) having the implanted shape when positioned into a targeted vascular defect of the subject,
the coil member (100) forming six sides when in the implanted shape,
**characterised in that** three sides (A, C, E) of the coil member (100) in the implanted shape each include a large loop (110) and another three of the sides (B, D, F) of the coil member (100) in the implanted shape each include two or more small loops (120).

2. The vaso-occlusive implant (10) of claim 1, wherein a diameter of the large loop (10) is at least 70% of a width of a corresponding one of the six sides (A, B, C, D, E, F) of the vaso-occlusive implant (10).

3. The vaso-occlusive implant (10) according to any one of the preceding claims, wherein a diameter of each of the two or more small loops (120) is less than 50% of a width of a corresponding one of the six sides (A, B, C, D, E, F) of the vaso-occlusive implant (10).

4. The vaso-occlusive implant (10) according to any one of the preceding claims, wherein the coil member (100) transitions from a first side to an immediately adjacent side for all sides of the coil member (100) in the implanted shape.

5. The vaso-occlusive implant (10) according to any one of the preceding claims, wherein the coil member (100) comprises a shape memory material.

6. The vaso-occlusive implant (10) of claim 5, wherein the shape memory material comprises a platinum tungsten alloy.

7. The vaso-occlusive implant (10) according to any one of the preceding claims, further comprising a tether (220) releasably secured to the coil member (100), wherein the coil member (100) is deliverable to the targeted vascular defect of the subject using the tether (220) and a pusher member (210).

8. The vaso-occlusive implant (10) of claim 7, wherein the tether (220) is compromisable to release the tether (220) from the coil member (100) at the targeted vascular defect of the subject.

9. The vaso-occlusive implant (10) according to any one of claims 7 or 8, wherein the tether (220) is compromisable mechanically, thermally, chemically or electrolytically.

10. The vaso-occlusive implant (10) according to any one of claims 7, 8, or 9, wherein the tether (220) includes a thermoplastic elastomer.

11. The vaso-occlusive implant (10) according to any of the preceding claims, wherein at least two of the six sides (A, B, C, D, E, F) are offset from one another by about 90 degrees.

12. The vaso-occlusive implant (10) according to any of the preceding claims, wherein the implanted shape of the coil member (100) is generally cubic.

13. The vaso-occlusive implant (10) according to any one of the preceding claims, wherein the coil member (100) in the elongated shape is deliverable to the targeted defect through an intraluminal device.

## Patentansprüche

1. Vasookklusives Implantat (10), umfassend:
ein Spulenelement (100), das von einer länglichen Form in eine implantierte Form expandierbar ist, wobei die längliche Form zur Abgabe in ein Subjekt dient und das Spulenelement (100) die implantierte Form aufweist, wenn es in einem Zielfgefäßdefekt des Subjekts positioniert wird,
wobei das Spulenelement (100) in der implantierten Form sechs Seiten bildet,
**dadurch gekennzeichnet, dass**
drei Seiten (A, C, E) des Spulenelements (100) in der implantierten Form jeweils eine große Schleife (110) einschließen und drei andere Seiten (B, D, F) des Spulenelements (100) in der implantierten Form jeweils zwei oder mehrere kleine Schleifen (120) einschließen.

2. Vasookklusives Implantat (10) nach Anspruch 1, wobei ein Durchmesser der großen Schlaufe (10) mindestens 70 % einer Breite einer entsprechenden der sechs Seiten (A, B, C, D, E, F) des vasookklusiven Implantats (10) beträgt.

3. Vasookklusives Implantat (10) nach einem der vorstehenden Ansprüche, wobei ein Durchmesser jeder der zwei oder mehreren kleinen Schleifen (120) weniger als 50 % einer Breite einer entsprechenden der sechs Seiten (A, B, C, D, E, F) des vasookklusiven Implantats (10) beträgt.

4. Vasookklusives Implantat (10) nach einem der vorstehenden Ansprüche, wobei das Spulenelement (100) in der implantierten Form für alle Seiten des Spulenelements (100) von einer ersten Seite zu einer unmittelbar benachbarten Seite übergeht.

5. Vasookklusives Implantat (10) nach einem der vorstehenden Ansprüche, wobei das Spulenelement (100) ein Formgedächtnismaterial umfasst.

6. Vasookklusives Implantat (10) nach Anspruch 5, wobei das Formgedächtnismaterial eine Platin-Wolfram-Legierung umfasst.

7. Vasookklusives Implantat (10) nach einem der vorstehenden Ansprüche, das weiter eine lösbar am Spulenelement (100) befestigte Leine (220) umfasst, wobei das Spulenelement (100) unter Verwendung der Leine (220) und eines Schiebeelements (210) zum Zielgefäßdefekt des Patienten gebracht werden kann.

8. Vasookklusives Implantat (10) nach Anspruch 7, wobei die Leine (220) kompromittiert werden kann, um die Leine (220) am Zielgefäßdefekt des Patienten vom Spulenelement (100) zu lösen.

9. Vasookklusives Implantat (10) nach einem der Ansprüche 7 oder 8, wobei die Leine (220) mechanisch, thermisch, chemisch oder elektrolytisch kompromittiert werden kann.

10. Vasookklusives Implantat (10) nach einem der Ansprüche 7, 8 oder 9, wobei die Leine (220) ein thermoplastisches Elastomer einschließt.

11. Vasookklusives Implantat (10) nach einem der vorstehenden Ansprüche, wobei mindestens zwei der sechs Seiten (A, B, C, D, E, F) um etwa 90 Grad voneinander versetzt sind.

12. Vasookklusives Implantat (10) nach einem der vorstehenden Ansprüche, wobei die implantierte Form des Spulenelements (100) im Allgemeinen würfelförmig ist.

13. Vasookklusives Implantat (10) nach einem der vorstehenden Ansprüche, wobei das Spulenelement (100) in der länglichen Form durch eine intraluminale Vorrichtung an den Zieldefekt abgegeben werden kann.

## Revendications

1. Implant vaso-occlusif (10) comprenant :
un élément de bobine (100) extensible d'une forme allongée à une forme implantée, la forme allongée étant destinée à être administrée à un sujet, et l'élément de bobine (100) présentant la forme implantée lorsqu'il est positionné dans une anomalie vasculaire ciblée du sujet,
l'élément de bobine (100) formant six côtés lorsqu'il est dans la forme implantée,
**caractérisé en ce que**
trois côtés (A, C, E) de l'élément de bobine (100) dans la forme implantée incluent chacun une grande boucle (110) et trois autres côtés (B, D, F) de l'élément de bobine (100) dans la forme implantée incluent chacun deux ou plusieurs petites boucles (120).

2. Implant vaso-occlusif (10) selon la revendication 1, dans lequel un diamètre de la grande boucle (10) est au moins égal à 70 % d'une largeur d'un côté correspondant parmi les six côtés (A, B, C, D, E, F) de l'implant vaso-occlusif (10).

3. Implant vaso-occlusif (10) selon l'une quelconque des revendications précédentes, dans lequel un diamètre de chacune parmi les deux ou plusieurs petites boucles (120) est inférieur à 50 % d'une largeur d'un côté correspondant parmi les six côtés (A, B, C, D, E, F) de l'implant vaso-occlusif (10).

4. Implant vaso-occlusif (10) selon l'une quelconque des revendications précédentes, dans lequel l'élément de bobine (100) passe d'un premier côté à un côté immédiatement adjacent pour tous les côtés de l'élément de bobine (100) dans la forme implantée.

5. Implant vaso-occlusif (10) selon l'une quelconque des revendications précédentes, dans lequel l'élément de bobine (100) comprend un matériau à mémoire de forme.

6. Implant vaso-occlusif (10) selon la revendication 5, dans lequel le matériau à mémoire de forme comprend un alliage platine-tungstène.

7. Implant vaso-occlusif (10) selon l'une quelconque des revendications précédentes, comprenant en outre une attache (220) fixée de manière amovible à l'élément de bobine (100), dans lequel l'élément de bobine (100) peut être administré à l'anomalie vasculaire ciblée du sujet à l'aide de l'attache (220) et d'un élément poussoir (210).

8. Implant vaso-occlusif (10) selon la revendication 7, dans lequel l'attache (220) peut compromettre la libération de l'attache (220) de l'élément de bobine (100) au niveau de l'anomalie vasculaire ciblée du sujet.

9. Implant vaso-occlusif (10) selon l'une quelconque des revendications 7 ou 8, dans lequel l'attache (220) peut être compromise mécaniquement, thermiquement, chimiquement ou électrolytiquement.

10. Implant vaso-occlusif (10) selon l'une quelconque des revendications 7, 8 ou 9, dans lequel l'attache (220) inclut un élastomère thermoplastique.

11. Implant vaso-occlusif (10) selon l'une quelconque des revendications précédentes, dans lequel au moins deux des six côtés (A, B, C, D, E, F) sont décalés l'un de l'autre d'environ 90 degrés.

12. Implant vaso-occlusif (10) selon l'une quelconque des revendications précédentes, dans lequel la forme implantée de l'élément de bobine (100) est généralement cubique.

13. Implant vaso-occlusif (10) selon l'une quelconque des revendications précédentes, dans lequel l'élément de bobine (100) de forme allongée peut être administré à l'anomalie ciblée par l'intermédiaire d'un dispositif intraluminal.
